# EUROPEAN PATENT SPECIFICATION

(11) **EP 4 553 482 B1**
(45) Date of publication and mention of the grant of the patent: **11.03.2026**
(21) Application number: 24207706.3
(22) Date of filing: 21.10.2024
(51) Int. Cl.: G01N 1/44, G01N 23/223, G01N 33/00

(54) **CERTIFICATION PROCESS FOR A TANGIBLE ASSET**
ZERTIFIZIERUNGSVERFAHREN FÜR EIN GREIFBARES VERMÖGEN
PROCÉDÉ DE CERTIFICATION POUR UN BIEN TANGIBLE

(30) Priority: 10.11.2023 IT 202300023799
(43) Date of publication of application: 14.05.2025
(73) Proprietor: BIT4ID S.r.l., 80125 Napoli (IT)
(72) Inventor: Campegiani, Paolo, 80124 Napoli (IT); Pilla, Pierluigi, 80124 Napoli (IT)
(74) Representative: Garavelli, Paolo

(56) References cited:
- JP-A- 2018 142 471
- US-A1- 2003 194 053
- US-A1- 2011 253 783
- US-A1- 2018 095 045

## Description

The present invention refers to a certification process for a tangible asset, in particular to a certification process for an ion-implanted tangible asset.

The elements that contribute to determining the commercial value of a tangible asset are many: the one that contributes most to determining the value of the tangible asset itself is the possibility of reconnecting it, in a sufficiently certain way, to the author.

In this context, a process of authenticating works of art is known through the issuing of a legal document, such as a certificate of authenticity, which accompanies the sale and purchase of a tangible asset. This certificate of authenticity is regulated, in Italy, by Legislative Decree 22 January 2004 n. 42, Cultural Heritage and Landscape Code, and consists of a photographic copy portraying the work, on the back of which some specifications are shown such as: title, type of support (canvas, plywood, paper, etc.), size, technique and materials used, date and place of creation and name of the author. The certificate of authenticity can be made more detailed by reporting: a brief description of the work, the state of conservation, the print run with the indication of the existence or otherwise of copies and the related number of copies, the gallery stamp. This certificate is reproduced in at least two copies, one owned by the collector and the other by the authenticator for archival purposes.

It is necessary that the certificate of authenticity and its related tangible asset are related. To this end, an identification system is known based on the assignment of a serial number affixed to the back of the tangible asset and on its certificate of authenticity. In particular, the well-known identification system uses anti-counterfeiting holographic labels equipped with a progressive number, preset to be broken if a removal is attempted, and printed with colour gradations, making their reproduction difficult.

In this context, it is possible to deduce that problems regarding the authenticity of a tangible asset arise when the artist or author disappears, because reasonable doubts may arise about the authoritativeness and reliability of posthumous authenticators. Cases of uncertainty regarding the authenticity of a work or a tangible asset are numerous and open to lengthy disputes accompanied by calligraphic assessments of the signatures placed on the certificates of authenticity and on the works themselves.

Currently, the world market for works of art is worth around 15.2 billion dollars and in Italy it exceeds 100 million euros. The Italian jewellery and goldsmith market is worth around 7 billion euros. The luxury personal goods market in Italy is worth 17.2 billion euros. The data reported above constitute a significant indicator of the great potential of the market for works of art and of the economic advantages that the market would benefit from if there were a safe, reproducible and non-invasive anti-counterfeiting system or process for certifying a tangible asset.

Tamper-proof holographic labels are known as anti-counterfeiting devices or systems, and can be positioned on the back of the tangible asset and on the accompanying paper certificate. These holographic labels are easily reproducible, through the availability of low-cost reproduction equipment on the market at affordable prices or by contacting regulated non-counterfeiting suppliers available on the Internet.

XRF imaging techniques are known that are capable of carrying out completely non-destructive investigations and are therefore widely applied in biology, geology, materials sciences, etc. However, the interest of archaeologists and art historians in maintaining the integrity of the assets that have come down to us, has made this type of advanced technique among the most used in the investigation of cultural heritage. The investigation using X-ray spectroscopy techniques makes it possible to analyse, without taking any fragments from the sample, the components, i.e. the chemical elements, of a material, which are important for a correct identification or for the search for any degradation processes. For example, in the case of painted objects, such as paints or ceramics, imaging the elemental distribution can provide a wealth of information regarding the stages of the painting process or changes made over the years. In fact, the technique allows to reveal any details hidden beneath the visible surface of the object, as well as modifications made by the artist himself and the possible restorations to which the work, or artefact, has been subjected over the years. The analysis of a painted object therefore allows obtaining a profound vision of the artist's creative process or the state of conservation of the work, allowing in-depth studies on the nature of the materials used and the manufacturing technologies that led to the finished product. Furthermore, due to the results obtained during the analysis activity, scholars are able to deduce information regarding the social, commercial and cultural development of the civilizations of origin of the works and the cultural and technological evolution to which they have been subjected during the course of time.

These techniques are not used for the certification of authenticity of artistic goods, since they are not able to verify their authenticity and correspondence with the author.

Patent application US 2011/253783 discloses a method of authenticating an article containing a chemical marking agent, which is substantially inseparably enclosed in a marker as a carrier and contains selected chemical elements and/or compounds in the form of marker elements, in concentrations based on a predetermined encryption code.

It is clear that there is no known certification process for a tangible asset capable of effectively avoiding counterfeiting and certifying its authenticity without leaving visible traces on the tangible asset itself, and which provides a unique characterization of the tangible asset, through an ion implantation.

The object of the present invention is therefore solving the aforementioned prior art problems, by providing a process for certifying a tangible asset through surface implantation of ions of two accelerated chemical elements.

Another object of the present invention is providing a certification process for a tangible asset designed to certify its authenticity, effectively evading counterfeiting action.

A final object of the present invention is providing a unique identification code and obtaining a codified mapping of the tangible asset.

The above and other objects and advantages of the present invention, as will appear from the following description, are achieved with a certification process for a tangible asset as described in the independent claim.

Preferred embodiments and non-trivial variations of the present invention are the subject matter of the dependent claims.

It is understood that all attached claims form an integral part of this description.

The present invention will be better described by some preferred embodiments thereof, provided by way of example and not by way of limitation, with reference to the attached drawings, in which:

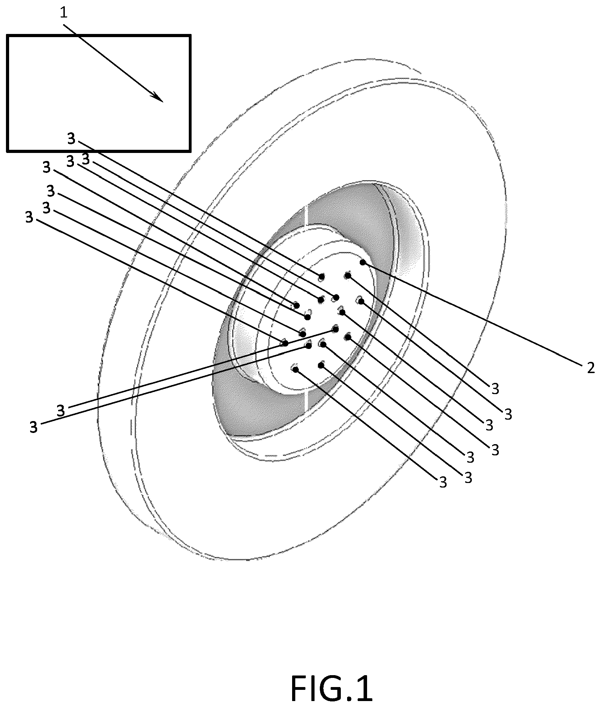

FIG. 1 and 2 show a three-dimensional view of a device for implementing a step of the process according to the present invention; and
FIG. 3 shows experimental evidence of the process according to the present invention.

Referring to the Figures, a preferred embodiment of the present invention is shown and described. It will be immediately obvious that countless variations and modifications can be made to what is described (for example relating to shape, dimensions, arrangements and parts with equivalent functionality) without departing from the scope of the invention as appears from the attached claims.

With reference to the Figures, the process of certifying a tangible asset such as, for example, a work of art, or a photographic work, or a manuscript, or a book, or another object of interest, is designed to guarantee, according to the present invention, the authenticity of the tangible asset by effectively avoiding counterfeiting and to certify its authenticity without leaving visible traces on the tangible asset itself. The certification process essentially includes the steps disclosed in independent claim 1.

Furthermore, the process includes a verification step *a posteriori* which consists of a comparison between the mapping of the relationships between the concentrations obtained by subjecting the tangible asset to steps a) to f) of the process with a previously codified mapping of the sample region of the tangible asset;
- if this comparison is successful, the mapping of the relationships between the concentrations coincides with the previously codified mapping, and the tangible asset has not been counterfeited;
- if this comparison is negative, the mapping of the relationships between the concentrations does not coincide with the previously codified mapping, and the tangible asset has been counterfeited.

In particular, the preliminary analysis is based on scanning the surface of the sample region of the tangible asset using a highly focused beam of X-rays with a few tens of microns. The result of this scan is a matrix of X-ray fluorescence spectra which are analysed individually, using appropriate analytical processes, in order to obtain the elemental maps of the samples. Each element of this matrix, in fact, is nothing other than the fluorescence spectrum acquired in the corresponding internal point of the sample region of the tangible asset. During the XRF scan, the sample slides horizontally along the movement for example, the size of the area to be analysed, the measurement of the interval that separates the individual XRF analyses making up the scan, the acquisition time of each fluorescence spectrum, etc.

Advantageously, during the surface implantation step, the relationship between the concentrations of the two chemical species is difficult to control exactly, but can be determined following surface implantation, offering the advantage of not requiring a measurement of the absolute concentrations of the two chemical species, but only their ratio, thus eliminating the need for complicated calibration processes of a control device during the control step.

As is known, ion implantation systems produce a pure, focused and high-energy ion beam, and propagate under vacuum, to avoid stopping it following interaction with air molecules. But the tangible asset cannot be subjected to a vacuum environment, therefore the known ion implantation systems and the related process cannot be applied to tangible assets. Consequently, the surface implantation in the sample region of the tangible asset of the two identified chemical species occurs through the production and extraction in air of a pure, high-energy ion beam, focused on the surface of the sample region of the tangible asset, using at least one extraction device 1 of the ion beam.

The extraction device 1 is centrally equipped with at least one flange 2 equipped with a plurality of holes 3, designed to facilitate the surface implantation of the two chemical species on the mapped sample region of the tangible asset (pattern).

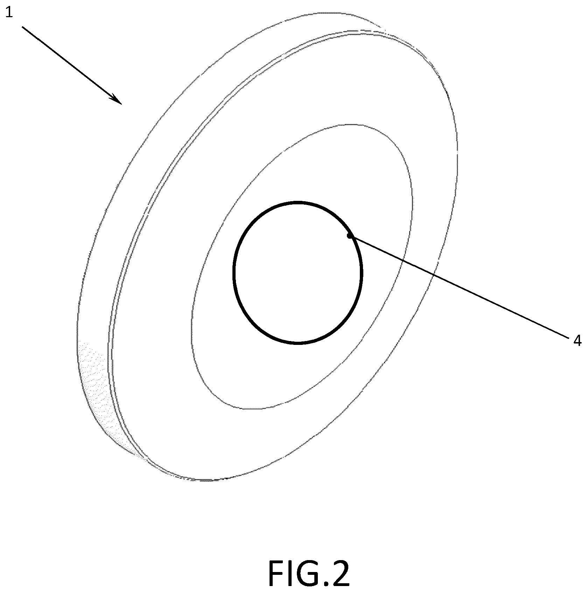

The surface implantation in the sample region of the tangible asset of the two chemical species consists of the following steps:
- preparation of the tangible asset and of the extraction device 1;
- production of an ion beam, using an ion accelerator;
- application of a coating film 4, such as, for example, Mylar, SiN, Evanhom, or the like, on the surface of the flange 2 of the extraction device 1, as shown in FIG. 2;
- exposing this surface covered by the coating film 4 to an accelerated ion beam;
- extraction of the ion beam in air, and obtaining an emerging ion beam;
- exposure of the sample region of the tangible asset to this emerging ion beam.

### EXPERIMENTAL EVIDENCE:

### PRELIMINARY ANALYSIS

Four types of material have been identified, such as copper for artistic goods made of metal, canvas for artistic goods such as paintings, ceramic and marble for artistic goods made respectively of these materials; in particular, these chemical compositions were considered: Copper = Pure copper Cu; Canvas = Aldehyde with CHO formyl group, Marble = 40% Limestone CaCO₃, 24% Silicon oxide SiO₂, 18% Calcium Carbonate CaCO₃ and 18% Dolomite MgCa(CO₃)₂, Ceramic = Chlorine Cl, Argon Ar, Potassium K, Calcium Ca, Scandium Sc, Vanadium V, Manganese Mn, Iron Fe, Cobalt Co, Nickel Ni.

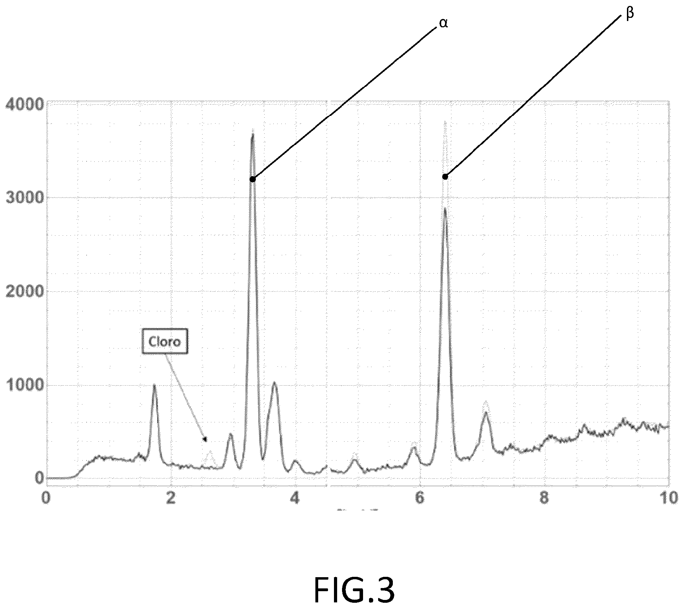

In particular, FIG. 3 shows the emission spectrum in the preliminary analysis step of a sample region of a tangible asset in ceramic material, using the X-ray fluorescence (XRF) technique, where the ordinate axis represents the number of net counts (counts) of each chemical element present in the sample region of the tangible asset and the x-axis represents the energy (keV) of the radiation emitted as function of the chemical elements present, allowing the identification of the chemical elements constituting the sample region.

In conclusion, Chlorine (and/or an ion with a close atomic number, such as Sulphur) appears to be the best candidate for surface implantation, comparing the radiation emitted before α and after β irradiation.

### ION BEAM PRODUCTION

In this step, an analysis was carried out of different usable ion beams and the related energies necessary to obtain the optimal implantation depth of the two identified chemical species.

The ion beams evaluated start from ²³Na to ⁵¹V and for each ion beam the energy necessary to overcome the coating film and the energy necessary to obtain an emerging ion beam with at least 1 MeV of energy was evaluated, considered sufficient to enable an implantation of the two chemical species identified on the sample region of the tangible asset.

The verification of the dose necessary for the detection of the implanted chemical species via XRF was carried out by implanting approximately 31016 ions of 35C1 in a ceramic sample placed under vacuum. The comparison of the XRF spectrum measured after implantation with that measured in the same area before it highlights the detectability of the implanted ions. The Chlorine ion was extracted from a composition of 50% by volume of Barium Chloride BaCl₂ and the remainder of Silver Ag, obtaining a mixture, then placed in a chamber heated to 70°C to reduce its water content. The mixture was installed in the ion source arranged at an upper end of the ion accelerator, a plurality of focusing and beam-selective elements induce the formation of an ion beam. Therefore, 35Cl+ ions were accelerated to 1.1 MeV with an average current yield of 280 nA (5% uncertainty, of which 3% reading and 2% for intensity fluctuations). ~3.5x1016 ions (6%, of which 1% conservative over time) were implanted in the sample in approximately 5h20'. The current was monitored periodically throughout the implantation. In conclusion, the ideal implantation depth was estimated to be equal to 0.6 µm.

### ION BEAM EXTRACTION

A plurality of materials and related thicknesses were evaluated to be used as a coating film to be applied on the surface of the flange of the extraction device, based on parameters such as, for example, energy loss, mechanical resistance, etc.

For this purpose, films of material were tested: 3.5 and 2 µm aluminized Mylar; 0.5 µm Silicon Nitride; and Evanhom of 2.5 µm.

The resistance tests were carried out both in vacuum and using the coating film as a confinement layer of the ion beam line with respect to the external atmosphere.

The film of Evanhom material presented the best resistance to the tests to which the aforementioned indicated materials were subjected. In particular, in one test an accelerated 32S6+ ion beam of 17.5 MeV was sent onto a film of 2.5 µm of EVANOHM material placed on the surface of the flange of the extraction device. The emerging beam, with a residual energy of 1 Mev, was revealed in the air through the luminescence produced by the beam itself, ionizing the helium atoms which were blown through a nozzle located immediately downstream of the extraction device.

The invention has the following advantages:
- allows certifying a tangible asset without carrying out any physical removal of material from the tangible asset;
- prevents altering the external appearance of the tangible asset, leaving no visible traces on the asset;
- allows proceeding with the aforementioned certification process in any step of conservation of the tangible asset;
- guarantees a stable and robust certification process, during possible exposure of the tangible asset to atmospheric agents, thermal cycles, restoration interventions, etc.;
- allows introducing a plurality of unique and stable parameters over time into the tangible asset, creating the digital footprint of the asset itself;
- provides a code representing the asset with a degree of complexity, depending on the robustness required for the tangible asset;
- allows certifying the asset at any time by verifying through non-contact and non-destructive analyses.

Some preferred embodiments of the present invention have been shown and described previously: obviously, numerous variations and modifications, functionally equivalent to the previous ones, which fall within the scope of the invention as highlighted in the attached claims, will be immediately evident to those skilled in the art.

## Claims

1. Certification process for at least one tangible asset including the steps of:
a) preparation of said tangible asset, and identification of at least one sample region of said tangible asset;
b) preliminary analysis and identification of the chemical composition of said sample region of said tangible asset, using the X-ray fluorescence, XRF, technique;
c) identification and choice of at least two unidentified chemical species in said sample region of said tangible asset during said preliminary analysis;
d) surface implantation in said sample region of said tangible asset of said two identified chemical species, in ratios between the concentrations characterizing a plurality of points of the sample region of the tangible asset, constituting by means of an absorbing medium a matrix of points, in which each point of said matrix of points is **characterized by** a certain relationship between the concentrations of said two chemical species, said surface implantation in the sample region of said tangible asset of the two identified chemical species occurring through the production and extraction in air of a pure ion beam, for example a high energy ion beam, and focused on the surface of the sample region of said tangible asset, by means of at least one extraction device (1) of said ion beam, said surface implantation comprising the following steps:
- preparation of said tangible asset and said extraction device (1);
- production of said ion beam, by means of an ion accelerator;
- application of at least one coating film (4) on the surface of at least one flange (2) of said extraction device (1);
- exposing said surface covered by said coating film (4) to an accelerated ion beam;
- extraction in air of said ion beam, and obtaining at least one emerging ion beam;
- exposure of the sample region of said tangible asset to said emerging ion beam;
e) generation of a mapping, pattern, of said relationships between concentrations, characterizing each point of said point matrix, depending on the position assumed by each point in said point matrix, enabling a unique characterization of the tangible asset;
f) production of a unique identification code associated with said mapping of the sample region of said tangible asset, depending on the nature of said two chemical species, and obtaining a codified mapping.

2. Process according to the previous claim, **characterized in that** it comprises a verification step *a posteriori,* comprising a comparison between the mapping of the relationships between the concentrations obtained by subjecting said tangible asset to said steps from a) to f) of said process with said codified mapping of the sample region of said tangible asset, and comparison between said mapping of the relationships between concentrations with said codified mapping of the sample region of said tangible asset:
- if said comparison is successful, said mapping between the relationships between the concentrations coincides with the codified mapping, and said tangible asset has not been counterfeited;
- if this comparison is negative, the mapping of the relationships between the concentrations does not coincide with the previously codified mapping, and the tangible asset has been counterfeited.

3. Process according to the previous claim, **characterized in that** said flange (2) of said extraction device (1) is equipped with a plurality of holes (3), designed to facilitate said superficial implantation of said two chemical species on the sample region of said mapped tangible asset, pattern.

## Patentansprüche

1. Zertifizierungsverfahren für mindestens einen materiellen Vermögenswert, einschließlich der folgenden Phasen:
a) Vorbereitung des genannten Sachvermögens und Identifizierung mindestens eines Musterbereichs des genannten Sachvermögens;
b) Voranalyse und Identifizierung der chemischen Zusammensetzung der Probenregion des Sachvermögens mittels Röntgenfluoreszenzanalyse (RFA);
c) Identifizierung und Auswahl von mindestens zwei chemischen Spezies, die in der genannten Probenregion der Ware bei der vorläufigen Analyse nicht identifiziert wurden;
d) Oberflächenimplantation der beiden identifizierten chemischen Spezies in den Probenbereich des Produkts, in Konzentrationsverhältnissen, die mehrere Punkte des Probenbereichs charakterisieren, durch Bildung einer Punktmatrix mittels eines Absorbers, wobei jeder Punkt der Punktmatrix durch ein spezifisches Konzentrationsverhältnis der beiden chemischen Spezies gekennzeichnet ist, die Oberflächenimplantation der beiden identifizierten chemischen Spezies in den Probenbereich des Produkts erfolgt durch Erzeugung und Extraktion eines reinen Ionenstrahls, beispielsweise eines Hochenergie-Ionenstrahls, in Luft, der mittels mindestens einer Extraktionsvorrichtung (1) auf die Oberfläche des Probenbereichs fokussiert wird, die Oberflächenimplantation umfasst die folgenden Phasen:
- Herstellung des genannten Sachvermögens und der genannten Fördervorrichtung (1);
- Erzeugung des besagten Ionenstrahls mittels eines Ionenbeschleunigers;
- Aufbringen mindestens eines Beschichtungsfilms (4) auf die Oberfläche mindestens eines Flansches (2) der Absaugvorrichtung (1);
- die mit dem Beschichtungsfilm (4) beschichtete Oberfläche einem beschleunigten Ionenstrahl aussetzen;
- Extraktion des besagten Ionenstrahls in die Luft und Gewinnung von mindestens einem austretenden Ionenstrahl;
- Exposition des Probenbereichs des genannten materiellen Vermögenswerts gegenüber dem austretenden Ionenstrahl;
e) Erzeugung einer Abbildung bzw. eines Musters der genannten Beziehungen zwischen Konzentrationen, die jeden Punkt der genannten Punktmatrix als Funktion der Position jedes Punktes in der genannten Punktmatrix charakterisieren und eine eindeutige Charakterisierung des materiellen Vermögenswerts ermöglichen;
f) Herstellung eines eindeutigen Identifikationscodes, der mit der genannten Kartierung des Probenbereichs des materiellen Gutes verknüpft ist, basierend auf der Art der beiden chemischen Spezies, und Erhalt einer codierten Kartierung.

2. Verfahren nach dem vorhergehenden Anspruch, **dadurch gekennzeichnet, dass** es einen Nachprüfungsschritt umfasst, der aus einem Vergleich der Konzentrationsbeziehungskarte, die durch Unterwerfen des materiellen Gutes den Schritten a) bis f) des Verfahrens unterzogen wurde, mit der codierten Karte des Probenbereichs des materiellen Gutes und einem Vergleich dieser Konzentrationsbeziehungskarte mit der codierten Karte des Probenbereichs des materiellen Gutes besteht:
- wenn der Vergleich erfolgreich ist, stimmt die Zuordnung der Konzentrationsverhältnisse mit der kodifizierten Zuordnung überein, und das materielle Gut wurde nicht gefälscht;
- wenn dieser Vergleich ein negatives Ergebnis liefert, stimmt die Zuordnung der Beziehungen zwischen den Konzentrationen nicht mit der zuvor kodifizierten Zuordnung überein, und das materielle Gut wurde gefälscht.

3. Extraktionsvorrichtung (1) nach dem vorhergehenden Anspruch, **dadurch gekennzeichnet, dass** der Flansch (2) der Extraktionsvorrichtung (1) mit einer Vielzahl von Löchern (3) versehen ist, die die Oberflächenimplantation der beiden chemischen Spezies auf dem Probenbereich des abgebildeten materiellen Gutes bzw. Musters begünstigen.

## Revendications

1. Procédure de certification d'au moins un actif corporel comprenant les phases suivantes:
a) la préparation dudit actif corporel et l'identification d'au moins une région échantillonnante dudit actif corporel;
b) analyse préliminaire et identification de la composition chimique de ladite région échantillonnée dudit actif corporel, en utilisant la technique de fluorescence X, XRF;
c) l'identification et la sélection d'au moins deux espèces chimiques non identifiées dans ladite région échantillonnée dudit bien matériel lors de ladite analyse préliminaire;
d) implantation de surface dans ladite zone échantillonnée dudit bien matériel des deux espèces chimiques identifiées, selon des rapports entre les concentrations caractérisant une pluralité de points de la zone échantillonnée du bien matériel, par constitution, au moyen d'un absorbeur, d'une matrice de points, chaque point de ladite matrice étant **caractérisé par** un rapport spécifique entre les concentrations desdites deux espèces chimiques, ladite implantation de surface dans la zone échantillonnée dudit bien matériel des deux espèces chimiques identifiées étant réalisée par la production et l'extraction dans l'air d'un faisceau d'ions pur, par exemple un faisceau d'ions de haute énergie, et focalisé sur la surface de la zone échantillonnée dudit bien matériel, au moyen d'au moins un dispositif d'extraction (1) dudit faisceau d'ions, ladite implantation de surface comprenant les phases suivantes:
- préparation dudit actif corporel et dudit dispositif d'extraction (1);
- production dudit faisceau d'ions, au moyen d'un accélérateur d'ions;
- application d'au moins un film de revêtement (4), sur la surface d'au moins une bride (2) dudit dispositif d'extraction (1);
- exposer ladite surface revêtue dudit film de revêtement (4), à un faisceau d'ions accélérés;
- extraction dudit faisceau d'ions dans l'air et obtention d'au moins un faisceau d'ions émergent;
- exposition de la zone échantillonnée dudit actif tangible audit faisceau d'ions émergent;
e) génération d'une cartographie, d'un modèle, desdites relations entre les concentrations, caractérisant chaque point de ladite matrice de points, en fonction de la position assumée par chaque point dans ladite matrice de points, favorisant une caractérisation unique de l'actif tangible;
f) la production d'un code d'identification unique associé à ladite cartographie de la région échantillonnée dudit bien matériel, basé sur la nature desdites deux espèces chimiques, et l'obtention d'une cartographie codée.

2. Procédé selon la revendication précédente, **caractérisé en ce qu'**il comprend une étape de vérification *a posteriori,* consistant en une comparaison entre la cartographie des relations de concentration obtenue en soumettant ledit bien matériel aux étapes a) à f) dudit procédé avec ladite cartographie codée de la zone échantillonnée dudit bien matériel, et une comparaison entre ladite cartographie des relations de concentration avec ladite cartographie codée de la zone échantillonnée dudit bien matériel:
- si la comparaison est concluante, la correspondance entre les relations entre les concentrations coïncide avec la correspondance codifiée, et le bien tangible n'a pas été contrefait;
- si cette comparaison aboutit à un résultat négatif, la cartographie des relations entre les concentrations ne coïncide pas avec la cartographie précédemment codifiée, et le bien tangible a été contrefait.

3. Dispositif d'extraction (1) selon la revendication précédente, **caractérisé en ce que** ladite bride (2) dudit dispositif d'extraction (1) est équipée d'une pluralité de trous (3), conçus pour favoriser ladite implantation de surface desdites deux espèces chimiques sur la région échantillon du bien tangible cartographié, modèle.
